# EUROPEAN PATENT SPECIFICATION

(11) **EP 3 989 812 B1**
(45) Date of publication and mention of the grant of the patent: **11.12.2024**
(21) Application number: 20735704.7
(22) Date of filing: 24.06.2020
(51) Int. Cl.: A61B 5/08, G01N 33/497, B60K 28/06, G01J 3/28

(54) **MULTI WAVELENGTH BREATH ANALYZING SYSTEM AND METHOD**
SYSTEM UND VERFAHREN ZUR ANALYSE DER ATMUNG MIT MEHREREN WELLENLÄNGEN
SYSTÈME ET PROCÉDÉ D'ANALYSE D'HALEINE À LONGUEURS D'ONDE MULTIPLES

(30) Priority: 25.06.2019 SE 1950781
(43) Date of publication of application: 04.05.2022
(73) Proprietor: Senseair AB, 820 60 Delsbo (SE)
(72) Inventor: LJUNGBLAD, Jonas, 112 56 Stockholm (SE); HÖK, Bertil, 723 44 Västerås (SE); MARTIN, Hans, 824 74 Delsbo (SE); RÖDJEGÅRD, Henrik, 820 64 Näsviken (SE)
(74) Representative: Zacco Sweden AB
(86) International application number: PCT/SE2020/050657
(87) International publication number: WO 2020/263168

(56) References cited:
- US-A1- 2008 061 238
- US-A1- 2015 219 620
- ALAN WAYNE JONES ET AL: "Determination of ethanol in breath for legal purposes using a five-filter infrared analyzer: studies on response to volatile interfering substances", JOURNAL OF BREATH RESEARCH, vol. 2, no. 2, 1 June 2008 (2008-06-01), US, pages 026006, XP055731555, ISSN: 1752-7155, DOI: 10.1088/1752-7155/2/2/026006
- M FRANSSON ET AL: "Laboratory Evaluation of a New Evidential Breath-Alcohol Analyser Designed for Mobile Testing - the Evidenzer", MEDICINE, SCIENCE AND THE LAW, vol. 45, no. 1, 1 January 2005 (2005-01-01), GB, pages 61 - 70, XP055731728, ISSN: 0025-8024, DOI: 10.1258/rsmmsl.45.1.61
- "Encyclopedia of Forensic and Legal Medicine", 1 January 2016, ELSEVIER, ISBN: 978-0-12-800055-7, article A.W. JONES: "Alcohol: Breath Analysis", pages: 119 - 137, XP055731496, DOI: 10.1016/B978-0-12-800034-2.00011-2

## Description

### Field of the invention

The present invention relates to a breath analyzing system and method. In particular the invention relates to a breath analyzing system and method arranged to provide absorption information in at least two different wavelength bands and using the information from the wavelength bands to identify an unidentified substance from a set of preselected substances.

### Background of the invention

Breath analyzing equipment is becoming increasingly common, not at least in vehicles as a measure to detect and prevent driving under the influence of intoxicating substances. The breath analyzing equipment may be a stand-alone, even handheld, unit that gives a measured value of the content of a substance or substances in the driver's breath. Alternatively, breath analyzing equipment may be part of a system wherein also including equipment for identifying the driver and/or immobilizing the vehicle. Such breath analyzing equipment is typically permanently mounted in the vehicle and may be an integral part of the dashboard, for example. Breath analyzing equipment may also be stationary systems used to control access to a work area, a vehicle fleet depot or the like.

To provide a breath analyzer that has an appropriate sensitivity, is reliable and provides a reasonable fast analysis is far from trivial. This is especially true if the breath analyzing equipment should be able to detect a plurality of substances and not being disturbed by variation in moisture, CO₂ content etc. Breath analyzing equipment that fulfills these requirements are described in for example US7919754 and US9746454, hereby incorporated by reference.

Breath alcohol analyzers based on non-dispersive infrared (NDIR) sensors commonly operate in the 9.4-9.6 µm wavelength range, as exemplified in the above referred patents. In this wavelength range ethyl alcohol has a strong absorption band with minor cross sensitivity to other substances. There are, however, challenges related to signal resolution, availability of infrared sources and detectors with adequate performance, and bulky design related to the long optical paths required.

Operation in the 3.3-3.6 µm range offers several advantages but has been hampered by high cross sensitivity to many substances which may occur in breath due to endogenous or exogenous origin. Current state of the art breath alcohol analyzers operating in the 3.3-3.6 µm range are typically equipped with a chopper wheel with several optical filters to perform the analysis. US 4,268,751 discloses a breath alcohol analyzer with a chopper wheel with several optical filters and has the capability of distinguishing between ethyl alcohol and acetone by means of two narrow-band filters at 3.39 and 3.48 µm. The chopper wheel design, incorporating plurality of moving parts, is not suitable outside a laboratory environment. Additionally a multiplicity of narrow-band filters such as described in "Determination of ethanol in breath for legal purposes using a five-filter infrared analyzer: studies on response to volatile interfering substances", A.W. Jones et al., JOURNAL OF BREATH RESEARCH, vol. 2, no. 2, 1 June 2008 (2008-06-01), would be needed to discern between more substances than ethyl alcohol and acetone. This would make the detector even more complicated, costly and unsuited for vehicle mounted or handheld devices.

### Summary of the invention

The object of the invention is to provide a breath analyzing system and method of operation that overcomes the drawbacks of operating in the 9.4-9.6 µm wavelength range and is suitable for being used in the field.

This is achieved by the breath analyzing method as defined in claim 1, and the breath analysis apparatus as defined in claim 18.

The breath analysis apparatus according to the invention for non-dispersive breath analysis operating in a preselected wavelength range of an unidentified substance , the breath analysis apparatus comprises:
- a measuring cell comprising non-dispersive infrared elements , the measuring cell comprising:
   - a source configured to transmit an infrared beam and at least first detector (6) and a second detector of infrared radiation within said wavelength range,
   - at least two concave mirrors arranged to control an infrared beam from said source to traverse the cell multiple times, thereby extending the optical path well beyond the physical dimensions of the cell,
   - a first interference filter with a first characteristic transition wavelength combined with a first infrared detector arranged in the optical path and configured to transmit a first wavelength band within the preselected wavelength range through the filter to the first infrared detector while reflecting a second wavelength band within the preselected wavelength range to be passed on to a second infrared detector, thereby the first infrared detector is configured to generate a first absorption signal associated to the first wavelength band and the second infrared detector is configured to generate a second absorption signal associated to the second wavelength band, wherein the first and second wavelength bands are at least to a major part separated by a preselected transition wavelength, λ₁, in the wavelength range between 3.3 and 3.6 µm, and preferably between 3.4 and 3.5 µm.

According to the invention the breath analysis apparatus further comprises a control unit arranged to receive at least the first and second absorption signals, the control unit configured to determine an absorption comparative value representing a comparison at least between the absorption in the first wavelength band and the absorption in the second wavelength band and a total absorption value representing a total absorption in the combined first wavelength band and the second wavelength band, and to compare the absorption comparative value and the total absorption value with tabulated data for a preselected set of substances arranged with corresponding values, and identifying the unidentified substance as the substance from the set of preselected substances representing the best match in terms of the absorption comparative values and the total absorption values. According to an aspect of the invention the breath analysis apparatus further comprises an auxiliary sensor unit configured to identify the reception of a human breath sample by means of peak detection of at least one tracer gas and determining a tracer gas concentration value. The control unit is typically configured to also determine breath concentration value of the identified substance using the tracer gas concentration value.

The method according to the invention of identifying an unidentified substance from a set of preselected substances during breath analysis of a human breath sample in a measuring cell using non-dispersive spectroscopy in a preselected wavelength range comprises the steps of:
- recording at least a first signal from a first infrared detector and a second signal from a second infrared detector, wherein the first signal represents the absorption in a first wavelength band in the in the preselected wavelength range and the second signal represents the absorption in a second wavelength band in the preselected wavelength range, wherein the first and second wavelength bands are at least to a major part separated by a preselected transition wavelength, λₜ;
- determining an absorption comparative value representing a comparison of at least the absorption in the first wavelength band and the absorption in the second wavelength band; and
- determining a total absorption value representing a total absorption in the combined first wavelength band and the second wavelength band;
- comparing the absorption comparative value and the total absorption value with tabulated data for the preselected set of substances arranged with corresponding values, and
- identifying the unidentified substance as the substance from the set of preselected substances representing the best match in terms of the absorption comparative values and the total absorption values.

According to the invention the preselected transition wavelength of the measuring cell, λₜ, is given by transition wavelength of the first interference filter, λ₁, the first interference filter being a high-pass filter and transmitting wavelengths above the preselected transition wavelength, λₜ, to the first infrared detector and passing wavelengths below the preselected transition wavelength, λₜ, to at least the second infrared detector. Alternatively the first interference filter is a low-pass filter transmitting wavelengths below the preselected transition wavelength, λₜ, to the first infrared detector and passing wavelengths above the preselected transition wavelength, λₜ, to at least the second infrared detector.

According to an aspect of the invention the non-dispersive spectroscopy is infrared non-dispersive spectroscopy and the preselected wavelength range is 3.3 to 3.6 µm.

According to an aspect of the invention the method further comprises a step of identifying the reception of a human breath sample by means of peak detection of at least one tracer gas and determining a tracer gas concentration value. A breath concentration value of the identified substance may be determined using the tracer gas concentration value.

According to an aspect of the invention if the identified substance is not ethanol, an error indication is issued.

According to an aspect of the invention a subset from the set of preselected substances has been predefined and a further step of determining a breath concentration value of the identified substance wherein the tracer gas concentration value is utilized is performed only if the identified substance is one of the substances in the subset. The predefined subset typically comprises substances for which regulations defining a maximum allowed concentration in breath or blood exists.

According to an aspect of the invention the absorption comparative value is a ratio between the absorption in the first wavelength band and the absorption in the second wavelength band. According to an aspect of the invention the total absorption is the sum of the absorption in the first wavelength band and the second wavelength band normalized with the tracer gas concentration value.

According to an aspect of the invention the tabulated data for the preselected set of substances has been arranged as tensor elements with coordinates representing absorption comparative values and a total absorption values for respective substance, and the step of comparing and determining comprises arranging the absorption comparative value and the total absorption value of the unidentified substance as coordinates in a corresponding multidimensional tensor, and quantifying the distance from the coordinates of the unidentified substance to at least a portion of the substances in the set of preselected substances and selecting the closest substance as the identified substance. The distances may be quantified by calculating the magnitudes and directions of the multidimensional tensors.

According to an aspect of the invention if a deviation in magnitude and direction between the unidentified substance and the identified substance is larger than a predetermined value, a notification is issued that identification of the unidentified substance could not be performed.

According to an aspect of the invention the preselected transition wavelength, λₜ, is between 3.3 and 3.6 µm, and preferably between 3.4 and 3.5 µm.

According to an aspect of the invention the first wavelength band and the second wavelengths band overlaps partly.

According to an aspect of the invention a specific target substance has been preselected and by that selection a number of potential interfering substances are identified, and the selection of interferences filters, as well as the numbers of filters and detectors, has been performed to optimize the separation of the target substance from the identified potential interfering substances. The specific target substance is typically ethyl alcohol and the identified potential interfering substances includes at least one of the substances: methyl alcohol, acetone, isopropyl alcohol and 1-propanol

According to an aspect of the method according to invention of the of recording comprises recording a first signal from a first infrared detector provided with a first interference filter with a first characterizing transition wavelength, a second signal from a second infrared detector provided with a second interference filter with a second characterizing transition wavelength and a third signal from a third infrared detector, wherein the first signal represents the absorption in a first wavelength band, the second signal represents the absorption in a second wavelength band and the third signal represents the absorption in a third wavelength band in the preselected wavelength range, wherein the first and second wavelength bands are at least to a major part separated by a preselected first transition wavelength, λ₁, corresponding to the first transition wavelength associated with the first interference filter and the second and third wavelength bands are at least to major part separated by a preselected second transition wavelength, λ₂, corresponding to the second transition wavelength associated with the second interference filter; and
in the steps of determining the absorption comparative value and the total absorption value, the absorption values of the first, second and third wavelength bands are utilized.

According to an aspect of the invention the breath analysis apparatus further comprises:
- a second interference filter with a characteristic transition wavelength combined with the second infrared detector; and
- a third infrared detector, and the control unit is configured to:
   - record a first signal from a first infrared detector, a second signal from a second infrared detector and a third signal from a third infrared detector, wherein the first signal represents the absorption in a first wavelength band, the second signal represents the absorption in a second wavelength band and the third signal represents the absorption in a third wavelength band in the preselected wavelength range, wherein the first and second bands are at least to a major part separated by the preselected first transition wavelength, λ₁, and wherein the second and third bands are at least to a major part separated by the preselected second transition wavelength, λ₁, given by the characteristic transition wavelength of the second interference filter; and
   - to determine the absorption comparative value representing a comparison between the absorption in the first wavelength band, the absorption in the second wavelength band and the absorption in the third wavelength band and the total absorption value represents a total absorption in the combined first wavelength band, the second wavelength band and the third wavelength band.

Thanks to the invention it is possible to provide a breath analysis apparatus operating in the 3.3-3.6 µm range. Thereby the breath analysis apparatus for non-dispersive breath analysis can be made in a compact format due to a shorter optical path. It is further possible to use components such as radiation sources and detectors that are less costly than in higher wavelength ranges.

One advantage afforded by the present invention is to identify a substance from a set of preselected substances in a efficient manner and with equipment that is comparably simple, and therefore robust and less costly compared to prior art techniques.

One further advantage is that it is possible to provide an accurate and reproducible separation of the 3.3-3.6 µm wavelength range into a first and second wavelength band by the uses of only one interference filter.

One further advantage is that it is possible to identify and discern between ethyl alcohol (ethanol) and a number of known "disturbing substances" by dividing the 3.3-3.6 µm wavelength range into only a first and second wavelength band and compare the absorption values according to the invention.

In the following, the invention will be described in more detail, by way of example only, with regard to non-limiting embodiments thereof, reference being made to the accompanying drawings.

### Brief description of the drawings

Fig. 1 is a schematic illustration of a breath analysis apparatus according to the present invention;
Fig. 2 is a graph showing absorption of ethyl alcohol in the 3.3-3.6 µm wavelength range;
Fig. 3a is a graph showing interference filter characteristics with one interference filter with a cutoff wavelengths of 3.5 µm, 3b is a graph showing interference filter characteristics with two interference filter with a cutoff wavelengths of 3.5 µm 3.4 µm respectively, and 3c 3b is a graph showing bandpass interference filter characteristics with two interference filter with bandpass wavelengths centered around 3.35, 3.45 and 3.55 µm, respectively;
Fig. 4 is a graph illustrating the mapping of absorption characteristics of selected substances based on data of Table 1;
Fig. 5a-e are schematic graphs illustrated the timing pattern of breath related signals; and
Fig 6 is a flowchart of the method according to the present invention.

### Detailed description

Terms such as "top", "bottom", upper", lower", "below", "above" etc are used merely with reference to the geometry of the embodiment of the invention shown in the drawings and/or during normal operation of the helmet and are not intended to limit the invention in any manner.

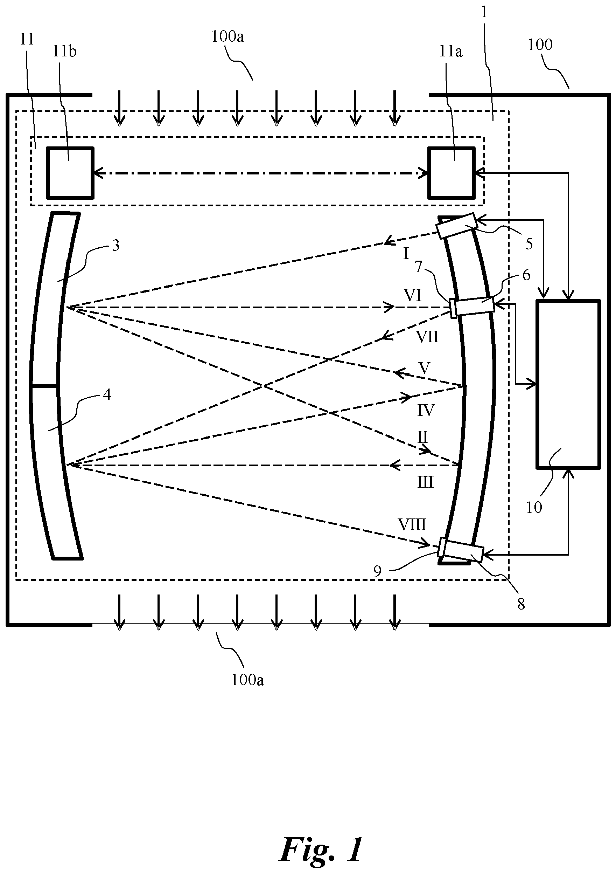

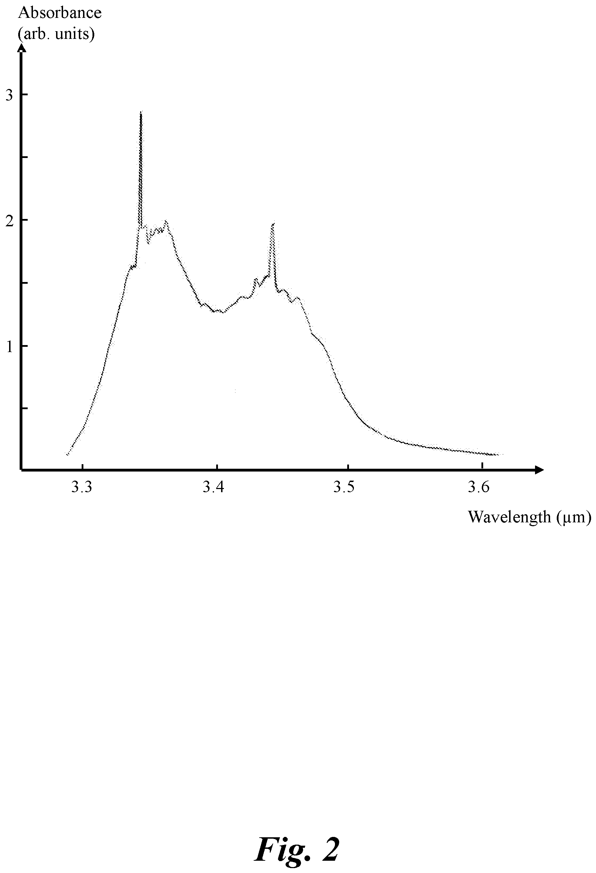

The breath analysis apparatus 100 according to the invention is schematically depicted in Fig. 1, and comprises a non-dispersive infrared (NDIR) measurement cell 1. The measuring cell 1 is configured to be operative in a preselected wavelength range, the preselected wavelength range typically governing the majority of other design parameters such as light source, detectors, filters, optical path etc. The measuring cell 1 according to the invention intended for detection of ethyl alcohol is optimized for a wavelength range of 3.3 to 3.6 µm. Fig 2 shows the absorption spectrum of ethyl alcohol in the wavelength range of 3.3-3.6 µm The double peaks indicate that the CH bonds are affected by the presence of an OR-group in one of the two carbon atoms of the molecule. In the spectra of other substances, it is possible to find other characteristics of the spectrum connecting to the molecular structure.

As apparent for the skilled person a breath analysis apparatus also comprises for example a housing, a mouthpiece, air ducts, a power unit and typically one or more fans and steerable vents. The basic designs of breath analysis apparatuses for different purposes, e.g. stationary, handheld and vehicle mounted are known in the art, for example from US7919754 and US9746454 and are therefore not depicted or elaborated on herein.

The breath analysis apparatus 100 is provided with an inlet portion 100a for receiving a breath sample from a human being and an outlet portion 100b for the breath sample to exit from the measuring cell 1. The breath sample is passing through the measuring cell 1 as indicated by the vertical arrows at the inlet and outlet portions 1a, 1b of the measuring cell 1. A beam of infrared radiation, indicated by dashed lines, is passing through the measuring cell in the transverse direction, starting from the light source 5 and being reflected multiple times I, II, III, IV, V, VI, VII VIII by concave mirrors 2, 3, 4 which are preferably designed and arranged according to the principles disclosed by J. U. White, J. Opt. Soc. Amer. 32 (1942), 285-288, a so called White cell configuration.

The light source 5 is an IR source being a black body emitter or light emitting diode, LED, operating within the preselected wavelength range. A suitable LED is made from multiple hetero-structures of III-V compounds with various constitution to operate as active layer and layers transparent to the emitted radiation. Both black body emitters, such as dedicated light bulbs and LEDs fulfill the necessary requirements for acting as suitable emitters in the system according to the invention.

The measuring cell 1 is provided with at least a first infrared detector 6 provided with a first interference filter 7 to precisely control the transmission and reflection of the infrared radiation. The transmitted portion of the infrared radiation is received by the first infrared detector and the reflected portion will after a number of reflections by the concave mirrors 2, 3, 4 be received by a second infrared detector 8. The infrared detectors/interference filters are typically provided on or near one of the concave mirrors 2, 3, 4. The second infrared detector 8 may be provided with a second interference filter 9 and further infrared detectors may be provided in the measuring cell 1. The interference filters 7, 9 typically include a transparent substrate with a multilayer thin film structure in which the layers have interchanging high and low index of refraction. The effect of this multilayer structure is to control the transmission and reflection properties of the filter. The transition wavelength, λₜ, is selectable by choice of materials and thicknesses of the multilayer structure. Typically interference filters suitable in the measuring cell 1 are high-pass or low-pass filters characterized by a transition wavelength λ above which the filter is transmitting (reflecting) radiation, and below which the filter is reflecting (transmitting). The transition wavelength may also be referred to as the cut-off wavelength. Alternatively the interference filters are bandpass filters which transmit wavelengths in a defined portion of the spectra and reflects outside of that defined portion. Suitable filters are commercially available. The first and second wavelength bands should to the major part not overlap, although a minor overlapping portion is acceptable. The selection of first and second wavelength band will be further discussed below.

According to one embodiment of the invention the first and second wavelength bands are completely separated. This may be achieved by using bandpass interference filters for the first and second interference filters with their transmitting portions sufficiently far apart. According to one embodiment of the invention the first and second wavelength bands overlap at the most 20 %, and preferably at the most 10%.

According to one embodiment of the invention the first interference filter 7 is a high-pass or low-pass filter with a characteristic transition wavelength, λ₁, and the second infrared detector 8 is not combined with any interference filter. The measuring cell 1 will facilitate detection in a first wavelength band and in a second wavelength band with only the first interference filter 7. The measuring cell 1 will be characterized by a preselected transition wavelength, λₜ, separating the first and second wavelength bands. The preselected transition wavelength, λₜ, is in this configuration given solely by the transition wavelength of the first interference filter, λ₁.

To achieve a separation of the preselected wavelength range into two bands using only one interference filter could be advantageous according to some aspect since it is a simple and robust design and high reproducibility between different measuring cells 1 could be expected. This is of particular importance since the method of the invention utilizes comparison with tabulated values in the performed analysis to identify an unidentified substance. This comparison is sensitive to variations in the cells preselected transition wavelength, λₜ, and therefore a simple, yet stable, configuration is advantageous.

According to one embodiment of the invention the measuring cell 1 is provided with a first interference filter 7 with a first transition wavelength, λ₁, a second infrared detector 8 provided with a second interference filters 9 with a second transition wavelength, λ₂, and a third infrared detector (not shown). Similar to what has been described above the measuring cell 1 will in this embodiment have a first, a second and a third wavelength band defining three regions in the preselected wavelength range 3.3 to 3.6 µm. The measuring cell 1 is characterized by a first preselected transition wavelength, λₜ₁, separating the first and second wavelength band and a second preselected transition wavelength, λₜ₂, separating the second and third wavelength band. The measuring cells first preselected transition wavelength, λt₁,is given by the transition wavelength of the first interference filter, λ₁, and the second preselected transition wavelength, λₜ₂, is given by the transition wavelength of the second interference filter, λ₂.

Detectors covering the 3.3-3.6 µm band are commercially available. Such detectors are typically photonic devices, e g photodiodes, having selective properties regarding responsivity to infrared radiation compared to e g thermopiles which are sensitive also to thermal effects due to convection or conduction.

Typical dimensions of the measuring cell 1 range between 14 x 16 x 6 mm and 90 x 50 x 30 mm with an optical path of 300 - 1000 mm depending on the actual application.

The measuring cell 1 may include an auxiliary sensor unit 11 with sensors 12 and control elements 13 with the objective of identifying a human breath and controlling the air flow through the measuring cell. These elements could preferably include means for the detection and quantification of a tracer gas, for example carbon dioxide or water vapor, which is inherently included in human breath.

The signals to and from the measuring cell 1 are controlled by an control unit 10 which is capable of real time signal processing and to execute preloaded and/or downloaded instructions. The control unit 10 may also be provided with communication means or connected to communications means for communication with for example a vehicle communication unit, a remote server etc. The communication may be wireless or wirebound.

As discussed in the background section a challenge in using the 3.3-3.6 µm range is the overlap of absorption signal between substances that could be present in a person's breath sample or in the environment wherein the sample is taken. The latter is particularly problematic if so called passive breath analysis is utilized, i.e. breath analysis performed without the person blowing into a mouthpiece. It has commonly been believed that a detailed spectrum analysis measurement, as exemplified in Fig. 2, is needed to discern for example ethyl alcohol from acetone or methyl alcohol. This requires expensive and complicated equipment. The inventors have realized that a careful selection of a limited number of wavelength bands within the 3.3-3.6 µm range is sufficient to identify and discern between a substantial number of substances that are either known volatile substances, target substances" or substances that are commonly known to disturb the measurements of target substances.

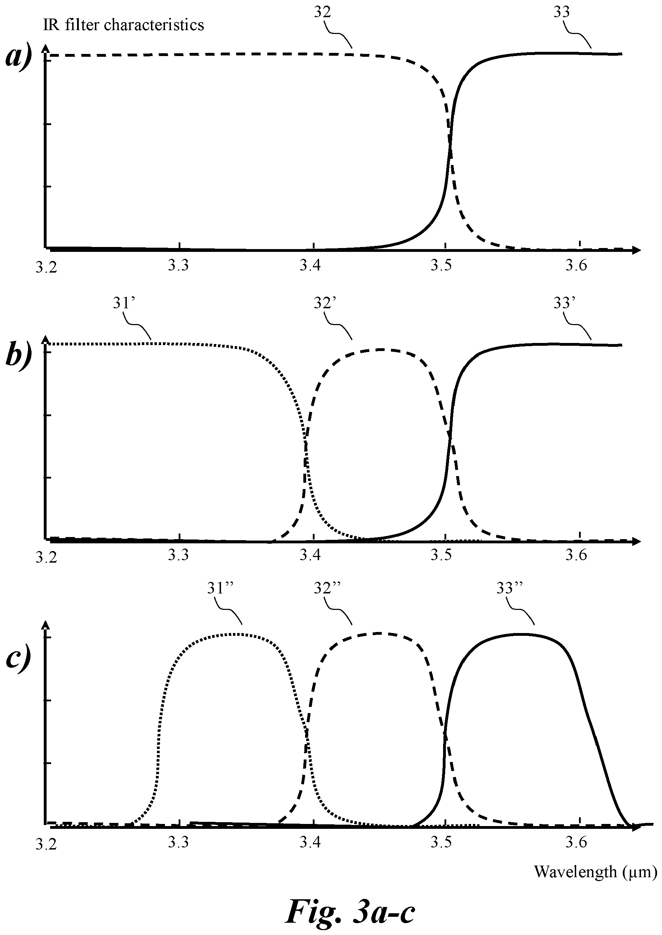

Fig. 3a shows the two spectra, 32 and 33 associated with the embodiment using only one interference filter, in this case a high-pass interference filter with a transition wavelength, λ₁, of 3.5 µm. The overlapping portion is depending on how sharp the transition is at the transition wavelength.

Fig. 3b shows the three spectra, 31', 32' and 33' associated with the embodiment a second interference filter and a further infrared detector (without filter), the second interference filter having a transition wavelength at 3.4 µm.

As discussed previously also bandpass interference filters could be used and Fig. 3c illustrates an embodiment wherein each three sensors are provided with bandpass interference filters: I the first interference filter 7 is configured to have the characteristic transmission illustrated by curve 33", i.e. a center wavelength of 3.55 µm and a width of 0.10 µm. Interference filter 9 is configured to have the characteristic transmission illustrated by curve 32", i.e. a center wavelength of 3.45 µm and a width of 0.10 µm. The third 9 is configured to have the characteristic transmission illustrated by curve 31", i.e. a center wavelength of 3.35 µm and a width of 0.10 µm.

Both the embodiment referring to Fig 3b and 3c give two transition wavelengths at 3.4 and 3.5 µm, dividing the wavelength range 3.3-3.6 into three wavelength bands.

In table 1 spectroscopic data from thirty substances, representing a preselected set of substances, have been combined with the filter spectra 31'/31", 32'/32" and 33'/33" to simulate absorption signals from the various wavelength bands, all normalized to ethanol (EtOH). The substances have been selected to represent gases which could be present in human breath for both endogenous and exogenous reasons. The spectroscopic data were obtained from Pacific Northwest National Laboratory, USA.

The six columns of Table 1 represent name of substance (left column), normalized absorption of the three wavelength bands (columns 2-4), the combined normalized absorption of the bands 3.4-3.5 µm and 3.5-3.6 µm (column 5), and the ratio between the absorption of the bands (column 6).

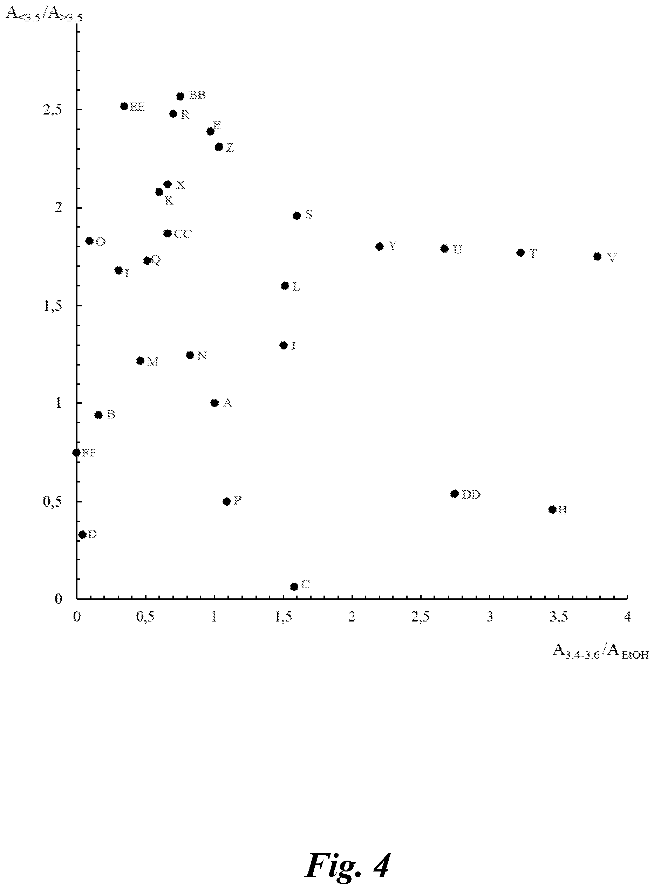

Fig. 4 is a graph using the data in column 5 in table 1 as x-axis and column 6 as y-axis. Each substance is represented by their coordinates in this two-dimensional graph. The coordinates are uniquely related to any one of the substances, although some of them are clustering in certain areas. To each coordinate a tensor can assigned and the magnitude and direction of the tensor is a unique property of each one of the listed substances and can be used to identified an unidentified substance. With the objective of identifying a certain unidentified substance, for example ethyl alcohol, its closest neighbors in the graph are isopropyl alcohol and methyl alcohol. Even though the magnitudes of their respective tensors do not differ very much, they may be distinguished from each other by different directions in the two-dimensional graph. Conversely, the tensors of 1-propanol and pentane have almost the same direction as that of ethyl alcohol, but their magnitudes differ significantly.

The embodiment described in relation to figures 1 and 3a-b utilizing one interference filter provides absorption signals which may directly be compared to the graph of Fig. 4. From absorption signals from a breath sample comprising one or more unidentified substances, its coordinates can be calculated and compared with the library obtained from columns 5 and 6 of table 1. It is thus possible to identify a substance by its coordinates by measuring the distance between the coordinates of the unidentified sample and any of the substances in the library. Zero distance means unambiguous identification. A finite distance could either mean that more than one substance is present in the sample, or that there is a remaining measurement error. By taking more samples, errors can be reduced.

The embodiment in relation to Fig 3c utilizing three wavelength bands is an extension by which a third dimension is added to the graph of Fig. 4. This implementation is useful to avoid the tendency of coordinate clustering illustrated in the graph of Fig. 4, and to improve the reliability of substance identification.

These embodiments are illustrating the basic principle of using a multidimensional property to be represented as a tensor having the rank of the actual dimensionality of the implementation, the first and second embodiment having rank two and three, respectively.

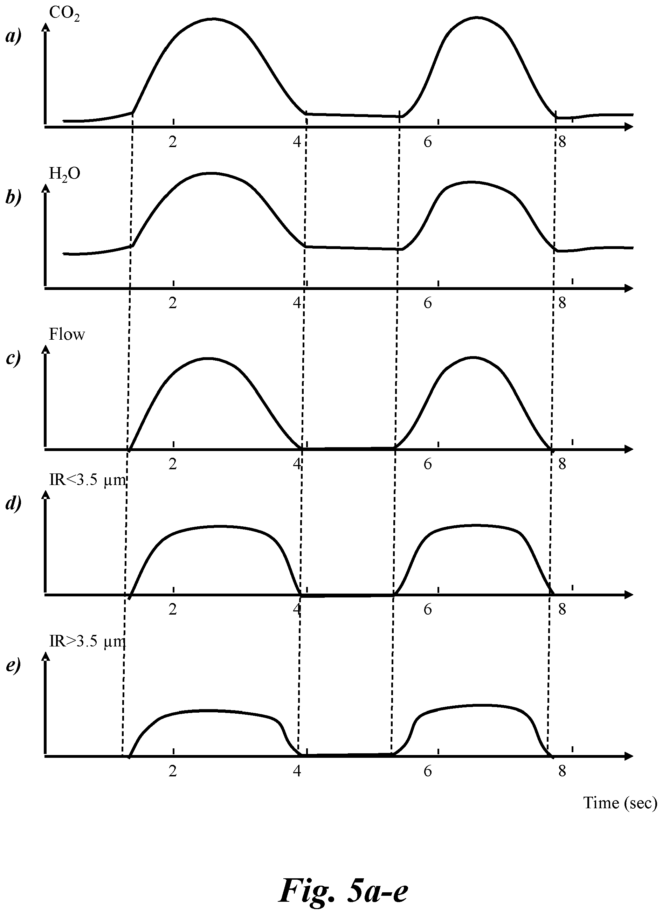

Fig. 5a-e shows a typical timing pattern of signals from two consecutive breath samples obtained from the auxiliary sensor unit 11, see Fig. 1 and from the measuring cell 1. Peaks of carbon dioxide CO₂ (a), water vapor H₂O (b), air flow (c), and the NDIR bands below (d) and above 3.5 µm (e) occur almost simultaneously. By recording these signals their origin from a human breath can be established. The signal baselines between breaths provide important information concerning eventual background concentration of any of the key substances.

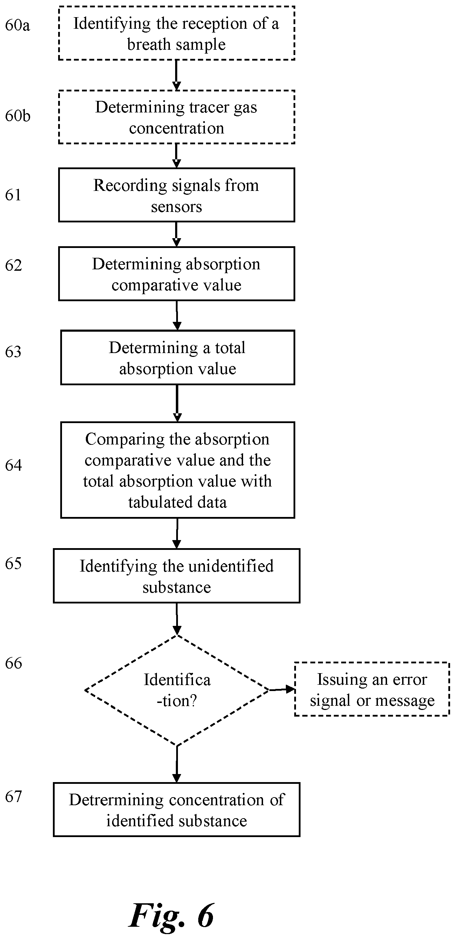

The method of identifying an unidentified substance from a set of preselected substances is illustrated in the flowchart of Fig. 6. The method is performed during breath analysis of a human breath sample in a measuring cell according to the invention with a preselected wavelength range, and the method comprises the steps of:
61: recording at least a first signal from a first infrared detector provided with a first interference filter and second signal from a second infrared detector, wherein the first signal represents the absorption, *A₁*, in a first wavelength band in the in the preselected wavelength range and the second signal represents the absorption in a second wavelength band, *A₂*, in the preselected wavelength range, wherein the first and second wavelength bands are at least to a major part separated and a preselected transition wavelength, λₜ, is separating the first and second wavelength band
62: determining an absorption comparative value representing a comparison of at least the absorption, *A₁*, in the first wavelength band and the absorption, *A₂*, in the second wavelength band;
63: determining a total absorption value representing a total absorption in at least the combined first wavelength band and the second wavelength band;
64: comparing the absorption comparative value and the total absorption value with tabulated data for the preselected set of substances arranged with corresponding values;
65: identifying the unidentified substance as the substance from the set of preselected substances representing the best match in terms of the absorption comparative values and the total absorption values.

According to one embodiment the first infrared detector is provided with a first interference filter and the preselected transition wavelength, λ₁, is the transition wavelength of the first interference filter thereby transmitting wavelengths above/below the preselected transition wavelength, λ₁, to the first infrared detector and passing wavelengths below/above the preselected transition wavelength, λ₁, to at least the second infrared detector. If wavelengths above/below the transmission wavelength is transmitted or reflected by the first interference filter 7 depends on if a high-pass or a low-pass configuration is utilized.

According to one embodiment, if the identified substance is not a predetermined target substance, for example ethyl alcohol, the method comprises the additional step, step 66, of issuing an error signal or message. Such an error message could include instructions to the user to move closer to the detector, to use a mouthpiece, if the first attempt was using passive detection, for example.

Typically the method also comprises a step, step 60a, of identifying the reception of a human breath sample by means of peak detection of at least one tracer gas, for example carbon dioxide and/or water vapor and a step, step 60b, of determining a tracer gas concentration value. This is preferably performed utilizing the auxiliary sensor unit 11. The identification of the reception of a human breath sample is used as a trigger for the further steps, which is exemplified in Fig. 5. In a step 67 a breath concentration value of the identified substance may be determined using the tracer gas concentration value is utilized.

According to one embodiment the absorption comparative value is a ratio between the absorption in the first wavelength band and the absorption in the second wavelength band, for example the ratio: *A₁*/*A₂.*

According to one embodiment the total absorption is the sum, *A₁*+*A₂*, of the absorption in the first wavelength band and the second wavelength band preferably normalized with the tracer gas concentration value.

According to one embodiment a subset from the set of preselected substances has been predefined. The predefined subset could be a set of detectable substances for which legal regulations, limit values stipulated by an industrial standard, limits imposed by an employer or equivalent exists. Such substances will be referred to as substances for which a regulation defining a maximum allowed concentration in breath or blood exists. A further step of the method, step 67, comprises checking if the identified substance is one of the substances in the predefined subset. The step of determining a breath concentration value of the identified substance wherein the tracer gas concentration value is utilized may be performed only if the identified substance is in the subset.

According to one embodiment and with reference to the discussion referring to Table 1 and Fig. 4, the tabulated data for the preselected set of substances has been arranged as tensor elements with coordinates representing absorption comparative values and a total absorption values for respective substance The steps of comparing 64 and determining 65 comprises arranging the absorption comparative value and the total absorption value of the unidentified substance as coordinates in a corresponding multidimensional tensor, and quantifying the distance from the coordinates of the unidentified substance to at least a portion of the substances in the set of preselected substances and selecting the closest substance as the identified substance. Computationally this can be done by a number of known routines, for example transformation of the coordinates of the tensor to place the coordinates of the unidentified substance in origo and calculating the distances to the other substances. The distances may be quantified by calculating the magnitudes and directions of the multidimensional tensors.

According to one embodiment if a deviation in magnitude and direction between the unidentified substance and the identified substance is larger than a predetermined value, a notification is issued that identification of the unidentified substance could not be performed.

According to one embodiment the preselected transition wavelength ,λₜ, is between 3.3 and 3.6 µm, and preferably between 3.4 and 3.5 µm.

As discussed referring to Table 1 and Fig. 4 the choice of the characteristics of the interferences filters, as well as the numbers of filters (detectors) greatly effects the multidimensional separation of substances. According to one embodiment a specific target substance is identified, for example ethyl alcohol, and the selection of interferences filters, as well as the numbers of filters, is done to optimize the separation of the target substance from known "close neighbors". According to one embodiment the specific target is ethyl alcohol and the filter parameters are selected to give a first preselected transition wavelength, λₜ₁, of 3.5 µm. Alternatively the filter parameters are selected to give a first preselected transition wavelength, λₜ, of 3.5 µm and a second preselected transition wavelength, λₜ₂, of 3.4 µm.

The control unit 10 of the breath analysis apparatus 100 according to the invention is configured to control and/or perform the method according to the invention. In particular the control unit 10 is configured to receive the signals (61) discussed in the method according to the invention and to perform the steps of determining an absorption comparative value 62, determining a total absorption value 63, comparing the absorption comparative value and the total absorption value with tabulated data 64 and identifying 65. The control unit 10 may further be configured to identify the reception of a human breath sample 60a and to determine tracer gas concentration value 60b.

According to one embodiment of the breath analysis apparatus 100 according to the invention, the first interference filter 7 is arranged in the optical path and configured to transmit a first wavelength band within the preselected wavelength range through the filter to be passed on to the first infrared detector 6 while reflecting a second wavelength band within the preselected wavelength range to be passed on to a second infrared detector 8, thereby the first infrared detector is configured to generate a first absorption signal, *A₁*, corresponding to the first wavelength band and the second infrared detector is configured to generate a second absorption signal, *A₂*, corresponding to the second wavelength band. The control unit 10 is arranged to receive at least the first and second absorption signals and to output a result indicating the quantified presence or absence of the unidentified substance within the breath sample. The control unit 10 is configured to determine an absorption comparative value representing a comparison at least between the absorption in the first wavelength band and the absorption in the second wavelength band and a total absorption value representing a total absorption in at least the combined first wavelength band and the second wavelength band, and to compare the absorption comparative value and the total absorption value with tabulated data for a preselected set of substances arranged with corresponding values, and identifying the unidentified substance as the substance from the set of preselected substances representing the best match in terms of the absorption comparative values and the total absorption values.

The embodiments described above are to be understood as illustrative examples of the system and method of the present invention. It will be understood that those skilled in the art that various modifications, combinations and changes may be made to the embodiments. In particular, different part solutions in the different embodiments can be combined in other configurations, where technically possible.

| ***Substance*** | ***Ref*** | ***3.30-3.40 µm*** | ***3.40-3.50 µm*** | ***3.50-3.60 µm*** | ***A_{3.4-3.6}*/*A _{EtOH}*** | ***A_{<3.5}*/*A_{>3.5}*** |
|---|---|---|---|---|---|---|
| Acetone | B | 0.37 | 0.15 | 0.17 | 0.16 | 0.94 |
| Acetaldehyde | C | 0.16 | 0.19 | 2.96 | 1.58 | 0.06 |
| Benzene | D | 0.12 | 0.02 | 0.06 | 0.04 | 0.33 |
| Butyl acetate | E | 1.96 | 1.36 | 0.57 | 0.97 | 2.39 |
| Carbon dioxide* | F | 0.00 | 0.00 | 0.00 | 0 | - |
| Carbon monoxide* | G | 0.00 | 0.00 | 0.00 | 0 | - |
| Diethyl ether | H | 1.77 | 2.16 | 4.74 | 3.45 | 0.46 |
| Ethanol (EtOH) | A | 1.00 (norm) | 1.00 (norm) | 1.00 (norm) | 1.0 (norm) | 1.0 (norm) |
| Ethyl acetate | I | 1.07 | 0.37 | 0.22 | 0.30 | 1.68 |
| 1-propanol | J | 1.45 | 1.69 | 1.30 | 1.50 | 1.30 |
| Ethyl benzene | K | 1.29 | 0.81 | 0.39 | 0.60 | 2.08 |
| Ethyl tert-butyl ether | L | 3.00 | 1.86 | 1.16 | 1.51 | 1.60 |
| Furfuryl alcohol | M | 0.26 | 0.50 | 0.41 | 0.46 | 1.22 |
| Isopropyl alcohol | N | 1.53 | 0.91 | 0.73 | 0.82 | 1.25 |
| Methane | O | 0.43 | 0.11 | 0.06 | 0.09 | 1.83 |
| Methyl alcohol | P | 0.79 | 0.73 | 1.46 | 1.09 | 0.50 |
| Methyl ethyl ketone | Q | 0.93 | 0.64 | 0.37 | 0.51 | 1.73 |
| m-Xylene | R | 1.03 | 0.99 | 0.40 | 0.70 | 2.48 |
| n-Butane | S | 2.42 | 2.12 | 1.08 | 1.60 | 1.96 |
| n-Heptane | T | 3.11 | 4.11 | 2.32 | 3.22 | 1.77 |
| n-Hexane | U | 2.89 | 3.43 | 1.92 | 2.67 | 1.79 |
| Octane | V | 3.32 | 4.80 | 2.75 | 3.78 | 1.75 |
| o-Xylene | X | 1.05 | 0.89 | 0.42 | 0.66 | 2.12 |
| Pentane | Y | 2.72 | 2.82 | 1.57 | 2.20 | 1.80 |
| Propane | Z | 2.17 | 1.43 | 0.62 | 1.03 | 2.31 |
| p-Xylene | BB | 1.17 | 1.08 | 0.42 | 0.75 | 2.57 |
| t-Butyl alcohol | CC | 1.98 | 0.86 | 0.46 | 0.66 | 1.87 |
| Tetrahydrofuran | DD | 1.93 | 1.93 | 3.55 | 2.74 | 0.54 |
| Toluene | EE | 0.57 | 0.48 | 0.19 | 0.34 | 2.52 |
| Water | FF | 0.0007 | 0.00003 | 0.00004 | 0.00004 | 0.75 |

Table 1 Absorption characteristics of selected substances relative to ethanol using three filters with center wavelengths 3.35, 3.45, and 3.55 µm, respectively defined by Fig. 3. Absorption data from Pacific Northwest National Laboratory, USA were used in the calculations. The entity ***A_{3.4-3.6}*/*A_{EtOH}*** is the total absorption within the wavelength range 3.4 -3.6 µm normalized to the corresponding absorption of ethanol. ***A_{<3.5}*/*A_{>3.5}*** is the ratio between the absorption in the wavelength bands below and above 3.5 µm. * Carbon dioxide and Carbon monoxide do not show any absorption in these wavelength ranges and are not included in the graph of Fig. 4.

## Claims

1. Method of identifying an unidentified substance from a set of preselected substances during breath analysis of a human breath sample in a measuring cell using non-dispersive spectroscopy in a preselected wavelength range, the method comprising the steps of:
- (61) recording at least a first signal from a first infrared detector and a second signal from a second infrared detector, wherein the first signal represents the absorption in a first wavelength band in the preselected wavelength range and the second signal represents the absorption in a second wavelength band in the preselected wavelength range, wherein the first and second wavelength bands are at least to a major part separated by a preselected transition wavelength, λₜ;
- (62) determining an absorption comparative value representing a comparison of at least the absorption in the first wavelength band and the absorption in the second wavelength band; and
- (63) determining a total absorption value representing a total absorption in the combined first wavelength band and the second wavelength band;
- (64) comparing the absorption comparative value and the total absorption value with tabulated data for the preselected set of substances arranged with corresponding values, and
- (65) identifying the unidentified substance as the substance from the set of preselected substances representing the best match in terms of the absorption comparative values and the total absorption values, and
- wherein the first infrared detector is provided with a first interference filter with a transition wavelength, λ₁, and the preselected transition wavelength of the measuring cell, λₜ, is given by transition wavelength of the first interference filter, λ₁, the first interference filter transmitting wavelengths above the preselected transition wavelength, λₜ, to the first infrared detector and passing wavelengths below the preselected transition wavelength, λₜ, to at least the second infrared detector, or
- wherein the first infrared detector is provided with a first interference filter with a transition wavelength, λ₁, and the preselected transition wavelength of the measuring cell, λₜ, is given by transition wavelength of the first interference filter, λ₁, the first interference filter transmitting wavelengths below the preselected transition wavelength, λₜ, to the first infrared detector and passing wavelengths above the preselected transition wavelength, λₜ, to at least the second infrared detector.

2. The method according to claim 1, wherein the non-dispersive spectroscopy is infrared non-dispersive spectroscopy and the preselected wavelength range is 3.3 to 3.6 µm.

3. The method according to any of the preceding claims, further comprising a step of identifying the reception of a human breath sample (60) by means of peak detection of at least one tracer gas and determining a tracer gas concentration value.

4. The method according to claim 3, further comprising a step of determining a breath concentration value of the identified substance (67) wherein the tracer gas concentration value is utilized.

5. The method according to any of the preceding claims, wherein, if the identified substance is not ethanol, an error indication is issued.

6. The method according to any of the preceding claims, wherein a subset from the set of preselected substances has been predefined and a further step of determining a breath concentration value of the identified substance wherein the tracer gas concentration value is utilized is performed only if the identified substance is one of the substances in the subset.

7. The method according to claim 6, wherein the predefined subset comprises substances for which regulations defining a maximum allowed concentration in breath or blood exists.

8. The method according to any of the preceding claims, wherein the absorption comparative value is a ratio between the absorption in the first wavelength band and the absorption in the second wavelength band.

9. The method according to claim 3, wherein the total absorption is the sum of the absorption in the first wavelength band and the second wavelength band normalized with the tracer gas concentration value.

10. The method according to any of the preceding claims, wherein the tabulated data for the preselected set of substances has been arranged as tensor elements with coordinates representing absorption comparative values and a total absorption values for respective substance, and the step of comparing (64) and determining (65) comprises arranging the absorption comparative value and the total absorption value of the unidentified substance as coordinates in a corresponding multidimensional tensor, and quantifying the distance from the coordinates of the unidentified substance to at least a portion of the substances in the set of preselected substances and selecting the closest substance as the identified substance.

11. The method according to claim 10, wherein the distances are quantified by calculating the magnitudes and directions of the multidimensional tensors.

12. The method according to claim 11, wherein, if a deviation in magnitude and direction between the unidentified substance and the identified substance is larger than a predetermined value, a notification is issued that identification of the unidentified substance could not be performed.

13. The method according to any of the preceding claims, wherein the preselected transition wavelength, λₜ, is between 3.3 and 3.6 µm, and preferably between 3.4 and 3.5 µm.

14. The method according to any of the preceding claims, wherein the first wavelength band and the second wavelengths band overlaps partly.

15. The method according to any of claims 1-14, wherein in the step of recording comprises recording a first signal from a first infrared detector provided with a first interference filter with a first characterizing transition wavelength, a second signal from a second infrared detector provided with a second interference filter with a second characterizing transition wavelength and a third signal from a third infrared detector, wherein the first signal represents the absorption in a first wavelength band, the second signal represents the absorption in a second wavelength band and the third signal represents the absorption in a third wavelength band in the preselected wavelength range, wherein the first and second wavelength bands are at least to a major part separated by a preselected first transition wavelength, λ₁, corresponding to the first transition wavelength associated with the first interference filter and the second and third wavelength bands are at least to major part separated by a preselected second transition wavelength, λ₂, corresponding to the second transition wavelength associated with the second interference filter; and
in the steps of determining the absorption comparative value (62) and the total absorption value (63), the absorption values of the first, second and third wavelength bands are utilized.

16. The method according to claim 1, wherein a specific target substance has been preselected and by that selection a number of potential interfering substances are identified, and the selection of interferences filters, as well as the numbers of filters and detectors, has been performed to optimize the separation of the target substance from the identified potential interfering substances.

17. The method according to claim 16, wherein a specific target substance is ethyl alcohol has been preselected and the identified potential interfering substances includes at least one of the substances: methyl alcohol, acetone, isopropyl alcohol and 1-propanol.

18. A breath analysis apparatus (100) for non-dispersive breath analysis operating in a preselected wavelength range of an unidentified substance, the breath analysis apparatus comprising:
- a measuring cell (1) comprising non-dispersive infrared elements , the measuring cell comprising:
- a source (5) configured to transmit an infrared beam;
- at least first detector (6) and a second detector (8) of infrared radiation within said wavelength range,
- at least two concave mirrors (2, 3, 4) arranged to control an infrared beam from said source to traverse the cell multiple times, thereby extending the optical path well beyond the physical dimensions of the cell (1),
- a first interference filter (7) with a first characteristic transition wavelength combined with a first infrared detector (6) arranged in the optical path of the measuring cell (1) and configured to transmit a first wavelength band within the preselected wavelength range through the filter to the first infrared detector (6) while reflecting a second wavelength band within the preselected wavelength range to be passed on to a second infrared detector (8), thereby the first infrared detector (6) is configured to generate a first absorption signal associated to the first wavelength band and the second infrared detector (8) is configured to generate a second absorption signal associated to the second wavelength band, wherein the first and second wavelength bands are at least to a major part separated by a preselected transition wavelength , λ₁, in the wavelength range between 3.3 and 3.6 µm, and preferably between 3.4 and 3.5 µm, the breath analysis apparatus (100) further comprising
- a control unit (10) arranged to receive at least the first and second absorption signals, the control unit configured to determine an absorption comparative value representing a comparison at least between the absorption in the first wavelength band and the absorption in the second wavelength band and a total absorption value representing a total absorption in the combined first wavelength band and the second wavelength band, and to compare the absorption comparative value and the total absorption value with tabulated data for a preselected set of substances arranged with corresponding values, and identifying the unidentified substance as the substance from the set of preselected substances representing the best match in terms of the absorption comparative values and the total absorption values., and wherein the preselected transition wavelength, λₜ, is the characteristic transition wavelength, λ₁, of the first interference filter (7), and wherein the first interference filter (7) is a high-pass filter transmitting wavelengths above the preselected transition wavelength, λₜ, to the first infrared detector and passing wavelengths below the preselected transition wavelength, λₜ, to at least the second infrared detector, or wherein the first interference filter (7) is a low-pass filter transmitting wavelengths above the preselected transition wavelength, λₜ, to the first infrared detector and passing wavelengths above the preselected transition wavelength, λₜ, to at least the second infrared detector.

19. The breath analysis apparatus according to claim 18, further comprising an auxiliary sensor unit (11) configured to identify the reception of a human breath sample by means of peak detection of at least one tracer gas and determining a tracer gas concentration value.

20. The breath analysis apparatus according to claim 18, wherein the control unit (10) is configured to determine breath concentration value of the identified substance using the tracer gas concentration value.

21. The breath analysis apparatus according to claim 18, wherein, if the identified substance is not ethanol, the control unit (10) is configured to issue an error indication.

22. The breath analysis apparatus according to claim 18, wherein the control unit (10) performs the determination of a breath concentration value of the identified substance only if the identified substance is one of the substances in a predefined subset from the set of preselected substances.

23. The breath analysis apparatus according to claim 22, wherein the predefined subset comprises substances for which regulation defining a maximum allowed concentration in breath or blood exists.

24. The breath analysis apparatus according to any of claims 18 to 22, wherein the absorption comparative value is a ratio between the absorption in the first wavelength band and the absorption in the second wavelength band.

25. The breath analysis apparatus according to claim 23, wherein the total absorption is the sum of the absorption in the first wavelength band and the second wavelength band normalized with the tracer gas concentration value.

26. The breath analysis apparatus according to any of claims 18 to 25, wherein the first wavelength band and the second wavelengths band overlaps partly.

27. The breath analysis apparatus according to any of claims 18 to 26, further comprising:
- a second interference filter (9) with a characteristic transition wavelength combined with the second infrared detector (8); and
- a third infrared detector; and
wherein the control unit is configured to:
- record a first signal from a first infrared detector, a second signal from a second infrared detector and a third signal from a third infrared detector, wherein the first signal represents the absorption in a first wavelength band, the second signal represents the absorption in a second wavelength band and the third signal represents the absorption in a third wavelength band in the preselected wavelength range, wherein the first and second bands are at least to a major part separated by the preselected first transition wavelength, λ₁, and wherein the second and third bands are at least to a major part separated by the preselected second transition wavelength, λ₁, given by the characteristic transition wavelength of the second interference filter (9); and
- to determine the absorption comparative value representing a comparison between the absorption in the first wavelength band, the absorption in the second wavelength band and the absorption in the third wavelength band and the total absorption value represents a total absorption in the combined first wavelength band, the second wavelength band and the third wavelength band.

## Patentansprüche

1. Verfahren zum Identifizieren einer nicht identifizierten Substanz aus einer Menge von vorausgewählten Substanzen während einer Atemluftanalyse einer Probe menschlicher Atemluft in einer Messzelle unter Verwendung nicht-dispersiver Spektroskopie in einem vorausgewählten Wellenlängenbereich, wobei das Verfahren die folgenden Schritte umfasst:
- (61) Aufzeichnen mindestens eines ersten Signals von einem ersten Infrarotdetektor und eines zweiten Signals von einem zweiten Infrarotdetektor, wobei das erste Signal die Absorption in einem ersten Wellenlängenband in dem vorausgewählten Wellenlängenbereich darstellt und das zweite Signal die Absorption in einem zweiten Wellenlängenband in dem vorausgewählten Wellenlängenbereich darstellt, wobei das erste und das zweite Wellenlängenband mindestens zu einem größeren Teil durch eine vorausgewählte Übergangswellenlänge, λₜ, getrennt sind;
- (62) Bestimmen eines Absorptionsvergleichswerts, der einen Vergleich von mindestens der Absorption in dem ersten Wellenlängenband und der Absorption in dem zweiten Wellenlängenband darstellt; und
- (63) Bestimmen eines Gesamtabsorptionswerts, der eine Gesamtabsorption in dem kombinierten ersten Wellenlängenband und dem zweiten Wellenlängenband darstellt;
- (64) Vergleichen des Absorptionsvergleichswerts und des Gesamtabsorptionswerts mit tabellarisierten Daten für die vorausgewählte Menge von Substanzen, die mit entsprechenden Werten angeordnet sind, und
- (65) Identifizieren der nicht identifizierten Substanz als die Substanz aus der Menge von vorausgewählten Substanzen, welche die beste Übereinstimmung bezüglich des Absorptionsvergleichswerts und der Gesamtabsorptionswerte darstellt, und
- wobei der erste Infrarotdetektor mit einem ersten Interferenzfilter mit einer Übergangswellenlänge, λ₁, bereitgestellt ist und die vorausgewählte Übergangswellenlänge der Messzelle, λₜ, durch die Übergangswellenlänge des ersten Interferenzfilters, λ₁, gegeben ist, wobei das erste Interferenzfilter Wellenlängen oberhalb der vorausgewählten Übergangswellenlänge, λₜ, an den ersten Infrarotdetektor überträgt und Wellenlängen unterhalb der vorausgewählten Übergangswellenlänge, λₜ, an mindestens den zweiten Infrarotdetektor weitergibt, oder
- wobei der erste Infrarotdetektor mit einem ersten Interferenzfilter mit einer Übergangswellenlänge, λ₁, bereitgestellt ist, und die vorausgewählte Übergangswellenlänge der Messzelle, λₜ, durch die Übergangswellenlänge des ersten Interferenzfilters, λ₁, gegeben ist, wobei das erste Interferenzfilter Wellenlängen unterhalb der vorausgewählten Übergangswellenlänge, λₜ, an den ersten Infrarotdetektor überträgt und Wellenlängen oberhalb der vorausgewählten Übergangswellenlänge, λₜ, an mindestens den zweiten Infrarotdetektor weitergibt.

2. Verfahren nach Anspruch 1, wobei die nicht-dispersive Spektroskopie eine nicht-dispersive Infrarotspektroskopie ist und der vorausgewählte Wellenlängenbereich 3,3 bis 3,6 µm beträgt.

3. Verfahren nach einem der vorhergehenden Ansprüche, ferner umfassend einen Schritt zum Identifizieren des Empfangs einer Probe (60) menschlicher Atemluft mittels Spitzenwerterkennung von mindestens einem Tracergas und Bestimmen eines Tracergaskonzentrationswerts.

4. Verfahren nach Anspruch 3, ferner umfassend einen Schritt zum Bestimmen eines Atemluftkonzentrationswerts der identifizierten Substanz (67), wobei der Tracergaskonzentrationswert verwendet wird.

5. Verfahren nach einem der vorhergehenden Ansprüche, wobei, wenn es sich bei der identifizierten Substanz nicht um Ethanol handelt, eine Fehleranzeige ausgegeben wird.

6. Verfahren nach einem der vorhergehenden Ansprüche, wobei eine Teilmenge aus der Menge von vorausgewählten Substanzen vordefiniert worden ist und ein weiterer Schritt zum Bestimmen eines Atemluftkonzentrationswerts der identifizierten Substanz, wobei der Tracergaskonzentrationswert verwendet wird, nur durchgeführt wird, wenn die identifizierte Substanz eine der Substanzen in der Teilmenge ist.

7. Verfahren nach Anspruch 6, wobei die vordefinierte Teilmenge Substanzen umfasst, für die es eine Vorschrift gibt, die eine maximal zulässige Konzentration in der Atemluft oder in dem Blut definiert.

8. Verfahren nach einem der vorhergehenden Ansprüche, wobei der Absorptionsvergleichswert ein Verhältnis zwischen der Absorption in dem ersten Wellenlängenband und der Absorption in dem zweiten Wellenlängenband ist.

9. Verfahren nach Anspruch 3, wobei die Gesamtabsorption die Summe der Absorption in dem ersten Wellenlängenband und dem zweiten Wellenlängenband normalisiert mit dem Tracergaskonzentrationswert ist.

10. Verfahren nach einem der vorhergehenden Ansprüche, wobei die tabellarisierten Daten für die vorausgewählte Menge von Substanzen als Tensorelemente mit Koordinaten angeordnet worden sind, die Absorptionsvergleichswerte und einen Gesamtabsorptionswert für eine jeweilige Substanz darstellen, und der Schritt zum Vergleichen (64) und zum Bestimmen (65) Anordnen des Absorptionsvergleichswerts und des Gesamtabsorptionswerts der nicht identifizierten Substanz als Koordinaten in einem entsprechenden mehrdimensionalen Tensor und Quantifizieren des Abstands von den Koordinaten der nicht identifizierten Substanz zu mindestens einem Teil der Substanzen in der Menge von vorausgewählten Substanzen und Auswählen der nächstgelegenen Substanz als die identifizierte Substanz umfasst.

11. Verfahren nach Anspruch 10, wobei die Abstände durch Berechnen der Größen und Richtungen der mehrdimensionalen Tensoren quantifiziert werden.

12. Verfahren nach Anspruch 11, wobei, wenn eine Abweichung in Größe und Richtung zwischen der nicht identifizierten Substanz und der identifizierten Substanz größer als ein vorbestimmter Wert ist, eine Meldung ausgegeben wird, dass eine Identifizierung der nicht identifizierten Substanz nicht durchgeführt werden konnte.

13. Verfahren nach einem der vorhergehenden Ansprüche, wobei die vorausgewählte Übergangswellenlänge, λₜ, zwischen 3,3 und 3,6 µm und vorzugsweise zwischen 3,4 and 3,5 µm liegt.

14. Verfahren nach einem der vorhergehenden Ansprüche, wobei das erste Wellenlängenband und das zweite Wellenlängenband sich teilweise überlappen.

15. Verfahren nach einem der Ansprüche 1-4, wobei der Schritt zum Aufzeichnen Aufzeichnen eines ersten Signals von einem ersten Infrarotdetektor, der mit einem ersten Interferenzfilter mit einer ersten kennzeichnenden Übergangswellenlänge bereitgestellt ist, eines zweiten Signals von einem zweiten Infrarotdetektor, der mit einem zweiten Interferenzfilter mit einer zweiten kennzeichnenden Übergangswellenlänge bereitgestellt ist, und eines dritten Signals von einem dritten Infrarotdetektor umfasst, wobei das erste Signal die Absorption in einem ersten Wellenlängenband darstellt, das zweite Signal die Absorption in einem zweiten Wellenlängenband darstellt und das dritte Signal die Absorption in einem dritten Wellenlängenband in dem vorausgewählten Wellenlängenbereich darstellt, wobei das erste und das zweite Wellenlängenband mindestens zu einem größeren Teil durch eine vorausgewählte erste Übergangswellenlänge, λ₁, getrennt sind, die der ersten Übergangswellenlänge entspricht, die dem ersten Interferenzfilter zugeordnet ist, und das zweite und das dritte Wellenlängenband mindestens zu einem größeren Teil durch eine vorausgewählte zweite Übergangswellenlänge, λ₂, getrennt sind, die der zweiten Übergangswellenlänge entspricht, die dem zweiten Interferenzfilter zugeordnet ist; und
in den Schritten zum Bestimmen des Absorptionsvergleichswerts (62) und des Gesamtabsorptionswerts (63) die Absorptionswerte des ersten, des zweiten und des dritten Wellenlängenbands verwendet werden.

16. Verfahren nach Anspruch 1, wobei eine spezifische Zielsubstanz vorausgewählt worden ist und durch diese Auswahl eine Anzahl von potentiellen Störsubstanzen identifiziert wird und die Auswahl von Interferenzfiltern sowie der Anzahlen von Filtern und Detektoren durchgeführt worden ist, um die Trennung der Zielsubstanz von den identifizierten potentiellen Störsubstanzen zu optimieren.

17. Verfahren nach Anspruch 16, wobei eine spezifische Zielsubstanz, bei der es sich um Ethylalkohol handelt, vorausgewählt worden ist und die identifizierten potentiellen Störsubstanzen mindestens eine der folgenden Substanzen beinhalten: Methylalkohol, Azeton, Isopropylalkohol und 1-Propanol.

18. Vorrichtung (100) zur Atemluftanalyse für die nicht-dispersive Atemluftanalyse, die in einem vorausgewählten Wellenlängenbereich einer unidentifizierten Substanz betrieben wird, wobei die Vorrichtung zur Atemluftanalyse Folgendes umfasst:
- eine Messzelle (1), umfassend nicht-dispersive Infrarotelemente, wobei die Messzelle Folgendes umfasst:
- eine Quelle (5), die dazu konfiguriert ist, einen Infrarotstrahl zu übertragen;
- mindestens einen ersten Detektor (6) und einen zweiten Detektor (8) für Infrarotstrahlung innerhalb des Wellenlängenbereichs,
- mindestens zwei konkave Spiegel (2, 3, 4), die dazu angeordnet sind, einen Infrarotstrahl von der Quelle so zu steuern, dass er die Zelle mehrere Male durchquert, wodurch sich der optische Weg weit über die physischen Abmessungen der Zelle (1) hinaus erstreckt,
- ein erstes Interferenzfilter (7) mit einer ersten charakteristischen Übergangswellenlänge, das mit einem ersten Infrarotdetektor (6) kombiniert ist, der in dem optischen Weg der Messzelle (1) angeordnet ist, und das dazu konfiguriert ist, ein erstes Wellenlängenband innerhalb des vorausgewählten Wellenlängenbereichs durch das Filter an den ersten Infrarotdetektor (6) zu übertragen, während ein zweites Wellenlängenband innerhalb des vorausgewählten Wellenlängenbereichs reflektiert wird, um an einen zweiten Infrarotdetektor (8) weitergegeben zu werden, wodurch der erste Infrarotdetektor (6) dazu konfiguriert ist, ein erstes Absorptionssignal, das dem ersten Wellenlängenband zugeordnet ist, zu erzeugen, und der zweite Infrarotdetektor (8) dazu konfiguriert ist, ein zweites Absorptionssignal, das dem zweiten Wellenlängenband zugeordnet ist, zu erzeugen, wobei das erste und das zweite Wellenlängenband mindestens zu einem größeren Teil durch eine vorausgewählte Übergangswellenlänge, λ₁, in dem Wellenlängenbereich zwischen 3,3 und 3,6 µm und vorzugsweise zwischen 3,4 und 3,5 µm getrennt sind, wobei die Vorrichtung (100) zur Atemluftanalyse ferner Folgendes umfasst:
- eine Steuereinheit (10), die dazu angeordnet ist, mindestens das erste und das zweite Absorptionssignal zu empfangen, wobei die Steuereinheit dazu konfiguriert ist, einen Absorptionsvergleichswert, der einen Vergleich mindestens zwischen der Absorption in dem ersten Wellenlängenband und der Absorption in dem zweiten Wellenlängenband darstellt, und einen Gesamtabsorptionswert, der eine Gesamtabsorption in dem kombinierten ersten Wellenlängenband und dem zweiten Wellenlängenband darstellt, zu bestimmen und den Absorptionsvergleichswert und den Gesamtabsorptionswert mit tabellarisierten Daten für eine vorausgewählte Menge von Substanzen, die mit entsprechenden Werten angeordnet sind, zu vergleichen und die nicht identifizierte Substanz als die Substanz aus der Menge von vorausgewählten Substanzen, welche die beste Übereinstimmung bezüglich des Absorptionsvergleichswerts und der Gesamtabsorptionswerte darstellt, zu identifizieren und wobei die vorausgewählte Übergangswellenlänge, λₜ, die charakteristische Übergangswellenlänge, λ₁, des ersten Interferenzfilters (7) ist und wobei das erste Interferenzfilter (7) ein Hochpassfilter ist, das Wellenlängen oberhalb der vorausgewählten Übergangswellenlänge, λₜ, an den ersten Infrarotdetektor überträgt und Wellenlängen unterhalb der vorausgewählten Übergangswellenlänge, λₜ, an mindestens den zweiten Infrarotdetektor weitergibt, oder wobei das erste Interferenzfilter (7) ein Tiefpassfilter ist, das Wellenlängen unterhalb der vorausgewählten Übergangswellenlänge, λₜ, an den ersten Infrarotdetektor überträgt und Wellenlängen oberhalb der vorausgewählten Übergangswellenlänge, λₜ, an mindestens den zweiten Infrarotdetektor weitergibt.

19. Vorrichtung zur Atemluftanalyse nach Anspruch 18, ferner umfassend eine Hilfssensoreinheit (11), die dazu konfiguriert ist, den Empfang einer Probe menschlicher Atemluft mittels Spitzenwerterkennung von mindestens einem Tracergas und Bestimmen eines Tracergaskonzentrationswerts zu identifizieren.

20. Vorrichtung zur Atemluftanalyse nach Anspruch 18, wobei die Steuereinheit (10) dazu konfiguriert ist, den Atemluftkonzentrationswert der identifizierten Substanz unter Verwendung des Tracergaskonzentrationswerts zu bestimmen.

21. Vorrichtung zur Atemluftanalyse nach Anspruch 18, wobei, wenn es sich bei der identifizierten Substanz nicht um Ethanol handelt, die Steuereinheit (10) dazu konfiguriert ist, eine Fehleranzeige auszugeben.

22. Vorrichtung zur Atemluftanalyse nach Anspruch 18, wobei die Steuereinheit (10) die Bestimmung eines Atemluftkonzentrationswerts der identifizierten Substanz nur dann durchführt, wenn die identifizierte Substanz eine der Substanzen in einer vordefinierten Teilmenge aus der Menge von vorausgewählten Substanzen ist.

23. Vorrichtung zur Atemluftanalyse nach Anspruch 22, wobei die vordefinierte Teilmenge Substanzen umfasst, für die es eine Vorschrift gibt, die eine maximal zulässige Konzentration in der Atemluft oder in dem Blut definiert.

24. Vorrichtung zur Atemluftanalyse nach einem der Ansprüche 18 bis 22, wobei der Absorptionsvergleichswert ein Verhältnis zwischen der Absorption in dem ersten Wellenlängenband und der Absorption in dem zweiten Wellenlängenband ist.

25. Vorrichtung zur Atemluftanalyse nach Anspruch 23, wobei die Gesamtabsorption die Summe der Absorption in dem ersten Wellenlängenband und dem zweiten Wellenlängenband normalisiert mit dem Tracergaskonzentrationswert ist.

26. Vorrichtung zur Atemluftanalyse nach einem der Ansprüche 18 bis 25, wobei das erste Wellenlängenband und das zweite Wellenlängenband sich teilweise überlappen.

27. Vorrichtung zur Atemluftanalyse nach einem der Ansprüche 18 bis 26, ferner umfassend:
- ein zweites Interferenzfilter (9) mit einer charakteristischen Übergangswellenlänge, das mit dem zweiten Infrarotdetektor (8) kombiniert ist; und
- einen dritten Infrarotdetektor; und
wobei die Steuereinheit zu Folgendem konfiguriert ist:
- Aufzeichnen eines ersten Signals von einem ersten Infrarotdetektor, eines zweiten Signals von einem zweiten Infrarotdetektor und eines dritten Signals von einem dritten Infrarotdetektor, wobei das erste Signal die Absorption in einem ersten Wellenlängenband darstellt, das zweite Signal die Absorption in einem zweiten Wellenlängenband darstellt und das dritte Signal die Absorption in einem dritten Wellenlängenband in dem vorausgewählten Wellenlängenbereich darstellt, wobei das erste und das zweite Band mindestens zu einem größeren Teil durch die vorausgewählte erste Übergangswellenlänge, λ₁, getrennt sind und wobei das zweite und das dritte Band mindestens zu einem größeren Teil durch die vorausgewählte zweite Übergangswellenlänge, λ₁, die durch die charakteristische Übergangswellenlänge des zweiten Interferenzfilter (9) gegeben ist, getrennt sind; und
- Bestimmen des Absorptionsvergleichswerts, der einen Vergleich zwischen der Absorption in dem ersten Wellenlängenband, der Absorption in dem zweiten Wellenlängenband und der Absorption in dem dritten Wellenlängenband darstellt, und des Gesamtabsorptionswerts, der eine Gesamtabsorption in dem kombinierten ersten Wellenlängenband, dem zweiten Wellenlängenband und dem dritten Wellenlängenband darstellt.

## Revendications

1. Procédé d'identification d'une substance non identifiée parmi un ensemble de substances présélectionnées lors de l'analyse de l'haleine d'un échantillon d'haleine humaine dans une cellule de mesure utilisant une spectroscopie non dispersive dans une plage de longueurs d'onde présélectionnée, le procédé comprenant les étapes consistant à :
- enregistrer (61) au moins un premier signal provenant d'un premier détecteur infrarouge et un deuxième signal provenant d'un deuxième détecteur infrarouge, le premier signal représentant l'absorption dans une première bande de longueurs d'onde dans la plage de longueurs d'onde présélectionnée et le deuxième signal représentant l'absorption dans une deuxième bande de longueurs d'onde dans la plage de longueurs d'onde présélectionnée, les première et deuxième bandes de longueurs d'onde étant au moins en grande partie séparées par une longueur d'onde de transition présélectionnée, λₜ ;
- déterminer (62) une valeur comparative d'absorption représentant une comparaison d'au moins l'absorption dans la première bande de longueurs d'onde et l'absorption dans la deuxième bande de longueurs d'onde ; et
- déterminer (63) une valeur d'absorption totale représentant une absorption totale dans la première bande de longueurs d'onde et la deuxième bande de longueurs d'onde combinées ;
- comparer (64) la valeur comparative d'absorption et la valeur d'absorption totale avec des données tabulées pour l'ensemble présélectionné de substances agencées avec des valeurs correspondantes, et
- identifier (65) la substance non identifiée comme la substance de l'ensemble de substances présélectionnées représentant la meilleure correspondance en termes de valeurs d'absorption comparatives et de valeurs d'absorption totales, et
- dans lequel le premier détecteur infrarouge est pourvu d'un premier filtre d'interférence avec une longueur d'onde de transition, λ₁, et la longueur d'onde de transition présélectionnée de la cellule de mesure, λₜ, est donnée par la longueur d'onde de transition du premier filtre d'interférence, λ₁, le premier filtre d'interférence transmettant des longueurs d'onde supérieures à la longueur d'onde de transition présélectionnée, λₜ, au premier détecteur infrarouge et transmettant des longueurs d'onde inférieures à la longueur d'onde de transition présélectionnée, λₜ, au moins au deuxième détecteur infrarouge, ou
- dans lequel le premier détecteur infrarouge est pourvu d'un premier filtre d'interférence avec une longueur d'onde de transition, λ₁, et la longueur d'onde de transition présélectionnée de la cellule de mesure, λₜ, est donnée par la longueur d'onde de transition du premier filtre d'interférence, λ₁, le premier filtre d'interférence transmettant des longueurs d'onde inférieures à la longueur d'onde de transition présélectionnée, λₜ, au premier détecteur infrarouge et transmettant des longueurs d'onde supérieures à la longueur d'onde de transition présélectionnée, λₜ, au moins au deuxième détecteur infrarouge.

2. Procédé selon la revendication 1, dans lequel la spectroscopie non dispersive est une spectroscopie non dispersive infrarouge et la plage de longueurs d'onde présélectionnée est de 3,3 à 3,6 µm.

3. Procédé selon l'une quelconque des revendications précédentes, comprenant en outre une étape d'identification de la réception d'un échantillon d'haleine humaine (60) au moyen d'une détection de pic d'au moins un gaz traceur et d'une détermination d'une valeur de concentration de gaz traceur.

4. Procédé selon la revendication 3, comprenant en outre une étape de détermination d'une valeur de concentration d'haleine de la substance identifiée (67) dans lequel la valeur de concentration de gaz traceur est utilisée.

5. Procédé selon l'une quelconque des revendications précédentes, dans lequel, si la substance identifiée n'est pas de l'éthanol, une indication d'erreur est émise.

6. Procédé selon l'une quelconque des revendications précédentes, dans lequel un sous-ensemble de l'ensemble de substances présélectionnées a été prédéfini et une étape supplémentaire de détermination d'une valeur de concentration dans l'haleine de la substance identifiée dans laquelle la valeur de concentration du gaz traceur est utilisée est effectuée uniquement si la substance identifiée est l'une des substances du sous-ensemble.

7. Procédé selon la revendication 6, dans lequel le sous-ensemble prédéfini comprend des substances pour lesquelles il existe des réglementations définissant une concentration maximale autorisée dans l'haleine ou le sang.

8. Procédé selon l'une quelconque des revendications précédentes, dans lequel la valeur d'absorption comparative est un rapport entre l'absorption dans la première bande de longueurs d'onde et l'absorption dans la deuxième bande de longueurs d'onde.

9. Procédé selon la revendication 3, dans lequel l'absorption totale est la somme de l'absorption dans la première bande de longueurs d'onde et dans la deuxième bande de longueurs d'onde normalisée avec la valeur de concentration du gaz traceur.

10. Procédé selon l'une quelconque des revendications précédentes, dans lequel les données tabulées pour l'ensemble présélectionné de substances ont été agencées sous forme d'éléments tenseurs avec des coordonnées représentant des valeurs d'absorption comparatives et des valeurs d'absorption totales pour la substance respective, et l'étape de comparaison (64) et de détermination (65) comprend l'agencement de la valeur d'absorption comparative et de la valeur d'absorption totale de la substance non identifiée sous forme de coordonnées dans un tenseur multidimensionnel correspondant, et la quantification de la distance entre les coordonnées de la substance non identifiée et au moins une partie des substances dans l'ensemble de substances présélectionnées et la sélection de la substance la plus proche comme substance identifiée.

11. Procédé selon la revendication 10, dans lequel les distances sont quantifiées en calculant les grandeurs et les directions des tenseurs multidimensionnels.

12. Procédé selon la revendication 11, dans lequel, si un écart de grandeur et de direction entre la substance non identifiée et la substance identifiée est supérieur à une valeur prédéterminée, une notification est émise indiquant que l'identification de la substance non identifiée n'a pas pu être effectuée.

13. Procédé selon l'une quelconque des revendications précédentes, dans lequel la longueur d'onde de transition présélectionnée, λₜ, est comprise entre 3,3 et 3,6 µm, et de préférence entre 3,4 et 3,5 µm.

14. Procédé selon l'une quelconque des revendications précédentes, dans lequel la première bande de longueurs d'onde et la deuxième bande de longueurs d'onde se chevauchent partiellement.

15. Procédé selon l'une quelconque des revendications 1 à 4, dans lequel l'étape d'enregistrement comprend l'enregistrement d'un premier signal provenant d'un premier détecteur infrarouge pourvu d'un premier filtre d'interférence avec une première longueur d'onde de transition caractérisante, d'un deuxième signal provenant d'un deuxième détecteur infrarouge pourvu d'un deuxième filtre d'interférence avec une deuxième longueur d'onde de transition caractérisante et d'un troisième signal provenant d'un troisième détecteur infrarouge, dans lequel le premier signal représente l'absorption dans une première bande de longueurs d'onde, le deuxième signal représente l'absorption dans une deuxième bande de longueurs d'onde et le troisième signal représente l'absorption dans une troisième bande de longueurs d'onde dans la plage de longueurs d'onde présélectionnée, dans lequel les première et deuxième bandes de longueurs d'onde sont au moins en majeure partie séparées par une première longueur d'onde de transition présélectionnée, λ₁, correspondant à la première longueur d'onde de transition associée au premier filtre d'interférence et les deuxième et troisième bandes de longueurs d'onde sont au moins en majeure partie séparées par une deuxième longueur d'onde de transition présélectionnée, λ₂, correspondant à la deuxième longueur d'onde de transition associée au deuxième filtre d'interférence ; et
dans les étapes de détermination de la valeur d'absorption comparative (62) et de la valeur d'absorption totale (63), les valeurs d'absorption des première, deuxième et troisième bandes de longueurs d'onde sont utilisées.

16. Procédé selon la revendication 1, dans lequel une substance cible spécifique a été présélectionnée et par cette sélection un certain nombre de substances interférentes potentielles sont identifiées, et la sélection de filtres d'interférences, ainsi que le nombre de filtres et de détecteurs, a été effectuée pour optimiser la séparation de la substance cible des substances interférentes potentielles identifiées.

17. Procédé selon la revendication 16, dans lequel une substance cible spécifique, l'alcool éthylique, a été présélectionnée et les substances interférentes potentielles identifiées comprennent au moins l'une des substances suivantes : l'alcool méthylique, l'acétone, l'alcool isopropylique et le 1-propanol.

18. Appareil d'analyse de l'haleine (100) pour une analyse de l'haleine non dispersive fonctionnant dans une plage de longueurs d'onde présélectionnée d'une substance non identifiée, l'appareil d'analyse de l'haleine comprenant :
- une cellule de mesure (1) comprenant des éléments infrarouges non dispersifs, la cellule de mesure comprenant :
- une source (5) configurée pour transmettre un faisceau infrarouge ;
- au moins un premier détecteur (6) et un deuxième détecteur (8) de rayonnement infrarouge dans ladite plage de longueurs d'onde,
- au moins deux miroirs concaves (2, 3, 4) agencés pour contrôler un faisceau infrarouge provenant de ladite source pour traverser la cellule plusieurs fois, prolongeant ainsi le trajet optique bien au-delà des dimensions physiques de la cellule (1),
- un premier filtre d'interférence (7) avec une première longueur d'onde de transition caractéristique combiné à un premier détecteur infrarouge (6) agencé dans le trajet optique de la cellule de mesure (1) et configuré pour transmettre une première bande de longueurs d'onde dans la plage de longueurs d'onde présélectionnée à travers le filtre au premier détecteur infrarouge (6) tout en réfléchissant une deuxième bande de longueurs d'onde dans la plage de longueurs d'onde présélectionnée à transmettre à un deuxième détecteur infrarouge (8), le premier détecteur infrarouge (6) étant configuré pour générer un premier signal d'absorption associé à la première bande de longueurs d'onde et le deuxième détecteur infrarouge (8) étant configuré pour générer un deuxième signal d'absorption associé à la deuxième bande de longueurs d'onde, les première et deuxième bandes de longueurs d'onde étant au moins en grande partie séparées par une longueur d'onde de transition présélectionnée, λ₁, dans la plage de longueurs d'onde comprise entre 3,3 et 3,6 µm, et de préférence entre 3,4 et 3,5 µm, l'appareil d'analyse de l'haleine (100) comprenant en outre :
- une unité de commande (10) conçue pour recevoir au moins les premier et deuxième signaux d'absorption, l'unité de commande étant conçue pour déterminer une valeur d'absorption comparative représentant une comparaison au moins entre l'absorption dans la première bande de longueurs d'onde et l'absorption dans la deuxième bande de longueurs d'onde et une valeur d'absorption totale représentant une absorption totale dans la première bande de longueurs d'onde et la deuxième bande de longueurs d'onde combinées, et pour comparer la valeur d'absorption comparative et la valeur d'absorption totale avec des données tabulées pour un ensemble présélectionné de substances agencées avec des valeurs correspondantes, et pour identifier la substance non identifiée comme la substance de l'ensemble de substances présélectionnées représentant la meilleure correspondance en termes de valeurs d'absorption comparatives et de valeurs d'absorption totales, et dans lequel la longueur d'onde de transition présélectionnée, λₜ, est la longueur d'onde de transition caractéristique, λ₁, du premier filtre d'interférence (7), et dans lequel le premier filtre d'interférence (7) est un filtre passe-haut transmettant des longueurs d'onde supérieures à la longueur d'onde de transition présélectionnée, λₜ, au premier détecteur infrarouge et passant des longueurs d'onde inférieures à la longueur d'onde de transition présélectionnée, λₜ, au moins au deuxième détecteur infrarouge, ou dans lequel le premier filtre d'interférence (7) est un filtre passe-bas transmettant des longueurs d'onde supérieures à la longueur d'onde de transition présélectionnée, λₜ, au premier détecteur infrarouge et transmettant des longueurs d'onde supérieures à la longueur d'onde de transition présélectionnée, λₜ, au moins au deuxième détecteur infrarouge.

19. Appareil d'analyse de l'haleine selon la revendication 18, comprenant en outre une unité de capteur auxiliaire (11) configurée pour identifier la réception d'un échantillon d'haleine humaine au moyen d'une détection de pic d'au moins un gaz traceur et pour déterminer une valeur de concentration de gaz traceur.

20. Appareil d'analyse de l'haleine selon la revendication 18, dans lequel l'unité de commande (10) est configurée pour déterminer la valeur de concentration de l'haleine de la substance identifiée à l'aide de la valeur de concentration de gaz traceur.

21. Appareil d'analyse de l'haleine selon la revendication 18, dans lequel, si la substance identifiée n'est pas de l'éthanol, l'unité de commande (10) est configurée pour émettre une indication d'erreur.

22. Appareil d'analyse de l'haleine selon la revendication 18, dans lequel l'unité de commande (10) effectue la détermination d'une valeur de concentration dans l'haleine de la substance identifiée uniquement si la substance identifiée est l'une des substances d'un sous-ensemble prédéfini de l'ensemble de substances présélectionnées.

23. Appareil d'analyse de l'haleine selon la revendication 22, dans lequel le sous-ensemble prédéfini comprend des substances pour lesquelles il existe une réglementation définissant une concentration maximale autorisée dans l'haleine ou le sang.

24. Appareil d'analyse de l'haleine selon l'une quelconque des revendications 18 à 22, dans lequel la valeur d'absorption comparative est un rapport entre l'absorption dans la première bande de longueurs d'onde et l'absorption dans la deuxième bande de longueurs d'onde.

25. Appareil d'analyse de l'haleine selon la revendication 23, dans lequel l'absorption totale est la somme de l'absorption dans la première bande de longueurs d'onde et la deuxième bande de longueurs d'onde normalisée avec la valeur de concentration du gaz traceur.

26. Appareil d'analyse de l'haleine selon l'une quelconque des revendications 18 à 25, dans lequel la première bande de longueurs d'onde et la deuxième bande de longueurs d'onde se chevauchent partiellement.

27. Appareil d'analyse de l'haleine selon l'une quelconque des revendications 18 à 26, comprenant en outre :
- un deuxième filtre d'interférence (9) avec une longueur d'onde de transition caractéristique combinée au deuxième détecteur infrarouge (8) ; et
- un troisième détecteur infrarouge ; et
dans lequel l'unité de commande est configurée pour :
- enregistrer un premier signal provenant d'un premier détecteur infrarouge, un deuxième signal provenant d'un deuxième détecteur infrarouge et un troisième signal provenant d'un troisième détecteur infrarouge, dans lequel le premier signal représente l'absorption dans une première bande de longueurs d'onde, le deuxième signal représente l'absorption dans une deuxième bande de longueurs d'onde et le troisième signal représente l'absorption dans une troisième bande de longueurs d'onde dans la plage de longueurs d'onde présélectionnée, dans lequel les première et deuxième bandes sont au moins en majeure partie séparées par la première longueur d'onde de transition présélectionnée, λ₁, et dans lequel les deuxième et troisième bandes sont au moins en majeure partie séparées par la deuxième longueur d'onde de transition présélectionnée, λ₁, donnée par la longueur d'onde de transition caractéristique du deuxième filtre d'interférence (9) ; et
- pour déterminer la valeur d'absorption comparative représentant une comparaison entre l'absorption dans la première bande de longueurs d'onde, l'absorption dans la deuxième bande de longueurs d'onde et l'absorption dans la troisième bande de longueurs d'onde et la valeur d'absorption totale représente une absorption totale dans la première bande de longueurs d'onde, la deuxième bande de longueurs d'onde et la troisième bande de longueurs d'onde combinées.
